# EUROPEAN PATENT SPECIFICATION

(11) **EP 2 064 245 B1**
(45) Date of publication and mention of the grant of the patent: **27.11.2013**
(21) Application number: 06791843.3
(22) Date of filing: 05.09.2006
(51) Int. Cl.: C07K 16/28, C07K 14/705, A61K 39/395

(54) **METHODS FOR BLOCKING THE INTERACTION BETWEEN NKP80 AND ITS LIGAND AICL**
VERFAHREN ZUM BLOCKIEREN DER WECHSELWIRKUNG ZWISCHEN NKP80 UND SEINEM LIGANDEN AICL
PROCÉDÉS DE BLOCAGE DE L'INTERACTION ENTRE NKP80 ET SON LIGAND AICL

(43) Date of publication of application: 03.06.2009
(73) Proprietor: Eberhard-Karls-Universität Tübingen, 72076 Tübingen (DE)
(72) Inventor: STEINLE, Alexander, 72070 Tübingen (DE); WELTE, Stefan, 63225 Langen (DE)
(74) Representative: Laufer, Gabriele
(86) International application number: PCT/EP2006/008647
(87) International publication number: WO 2008/028501

(56) References cited:
- EP-A- 1 219 637
- WO-A-2004/069183
- DEGLI-ESPOSTI MARIAPIA A ET AL: "Close encounters of different kinds: dendritic cells and NK cells take centre stage." NATURE REVIEWS. IMMUNOLOGY FEB 2005, vol. 5, no. 2, February 2005 (2005-02), pages 112-124, XP002433181 ISSN: 1474-1733
- WELTE STEFAN ET AL: "Mutual activation of natural killer cells and monocytes mediated by NKp80-AICL interaction." NATURE IMMUNOLOGY DEC 2006, vol. 7, no. 12, December 2006 (2006-12), pages 1334-1342, XP002433182 ISSN: 1529-2908
- RODA-NAVARRO P ET AL: "HUMAN KLRF1, A NOVEL MEMBER OF THE KILLER CELL LECTIN-LIKE RECEPTORGENE FAMILY: MOLECULAR CHARACTERIZATION, GENOMIC STRUCTURE, PHYSICAL MAPPING TO THE NK GENE COMPLEX AND EXPRESSION ANALYSIS" EUROPEAN JOURNAL OF IMMUNOLOGY, WEINHEIM, DE, vol. 30, no. 2, February 2000 (2000-02), pages 568-576, XP001062417 ISSN: 0014-2980

## Description

The present invention relates to methods for blocking the interaction between NKp80 and its ligands.

NKp80 represents an activating NK receptor of Natural Killer (NK) cells. NK cells and T cells are key effectors in immune responses. Originally, NK cells were considered mainly as innate immune effector cells spontaneously destroying infected or tumor cells. However, recent findings suggest an important role for NK cells also in the initiation and modulation of adaptive immune responses.

Unlike cytotoxic T lymphocytes (CTL), NK cells are capable of killing cancer and viral-infected cells without prior sensitization or immune memory. Activation and lysis of target cells is triggered mainly by natural cytotoxicity receptors (NCR) and NKG2D. However, additional activating receptors or costimulatory molecules also modulate NK cell activation.

To control their cytotoxic activity NK cells have two types of surface receptor, i.e. activating receptors that trigger killing by the NK cell and inhibitory receptors that inhibit activation and prevent NK cells from killing "healthy" cells. The NK activating receptors on the cell surface recognize proteins expressed on the surface of other cells like cancer or viral-infected cells and activate the NK to kill these target cells. Several types of activating receptors provide activation signals, including C-type lectin-like receptors. The "inhibitory receptors" are specific for MHC class I molecules, which explains why NK cells selectively kill target cells bearing low levels of MHC class I molecules.

In humans, a number of activating NK receptors including NKp30, NKp44, NKp46, NKp80 and NKG2D has been characterized. Only ligands of the NKG2D receptor have been described so far. The cellular ligands of the NK receptors NKp30, NKp44, NKp46, NKp80 are unknown and their identification would permit thorough understanding of NK cell biology.

NKp80, which has been reported to be NK cell-specific, stimulates NK cytoxicity and induces Ca**- influx in human NK cells upon triggering by appropriate antibodies (Vitale et al., "Identification of NKp80, a novel triggering molecule expressed by human NK cells", Eur. J. Immunol. 31, 233-242 (2001)).

Since NKp80, unlike NKG2D, does not contain charged amino acids in the transmembrane domain, an association with activating adaptor proteins seems unlikely. Furthermore, NKp80 does not contain any known activation motifs in the cytoplasmic sequence.

As mentioned above, activating receptors like NKp80 play an important role in the initiation and modulation of innate and adaptive immune responses.

In view of the above, there is a need in identifying cellular ligands of NKp80, not only in order to be able to characterize the effects of the ligands' binding to NKp80, but also to provide possible targets to affect their interaction.

Therefore, it is an object of the present invention to identify ligands of NKp80 and to provide substances to modulate the interaction between NKp80 and its ligands in order to treat and/or prevent diseases linked to an activation of NKp80 through its ligands.

According to the invention this object is achieved by identifying ligands of NKp80 on myeloid cells, in particular of AICL (Activation-induced C-type Lectin) as a ligand of NKp80.

Further, according to the invention this object is achieved by providing substances blocking the interactions between NKp80 and its ligands, in particular AICL.

Further, according to the invention this object is solved by the use of the substance blocking the interaction between NKp80 and its ligands, in particular AICL, for the manufacture of a medicament for the treatment or prevention of inflammatory diseases, in particular of autoimmune diseases, preferably of inflammatory rheumatic diseases like rheumatoid arthritis.

The object underlying the invention is completely solved in this way.

The inventors of the present invention have shown that myeloid cells express ligands of NKp80. They were further able to demonstrate binding of NKp80 expressed on NK cells to malignant myeloid cells via its ligands on the myeloid cells, as well as subsequent lysis of the myeloid cells due to the thus stimulated cytotoxicity of the NK cells against the myeloid cells.

In particular, it could be shown that AICL is a ligand of NKp80. Further, the inventors were able to show that AICL is a novel myeloid-specific, activating receptor expressed by monocytes, macrophages and granulocytes. In addition, they show that cross-linking of both NKp80 and AICL stimulates secretion of pro-inflammatory cytokines.

The inventors further showed that expression of AICL increases the susceptibility of myeloid cells to NK lysis by engaging NKp80.

As intended herein "substances blocking the interaction" relates to substances being capable to block or hamper the interaction between NKp80 and its ligands, in particular AICL and/or the effects associated with the interaction. Thus, according to the invention, not only substances that specifically block the binding of NKp80 by it ligands, in particular AICL, are suitable within the meaning of the present invention, i.e. substances, that - by binding either its ligands, e.g. AICL, or NKp80 - inhibit binding of the ligands to NKp80. In addition, within the meaning of the present invention, substances are suitable that bind to its ligands, e.g. AICL, or NKp80 and that do not block NKp80/ligand binding, but that inhibit the activating interaction between NKp80 and its ligands. For example, such substances can be antibodies directed against the ligands, e.g. AICL, or NKp80 or fragments thereof. In addition soluble ligands like soluble AICL or fragments thereof, being capable of binding to NKp80, can be used or soluble NKp80 or fragments thereof being capable of binding to the ligands can be used.

As intended herein "functional fragments" relate to substances that represent parts of major compounds, the parts still having one or more of the functional characteristics of the compounds they are derived from.

The finding, that AICL is specifically expressed on myeloid cells and that AICL is a ligand for NKp80 specifically enables the modulation of the interaction between AICL and NKp80 thereby providing an important tool in treating diseases in the course of which a constant stimulation of NK cells and myeloid cells causes chronic inflammatory reactions and release of inflammatory cytokines, as it is the case for, e.g., autoimmune disorders, and in particular inflammatory rheumatoid disorders like rheumatoid arthritis and related conditions.

The cause of rheumatoid arthritis is still unknown. Rheumatoid arthritis is an inflammatory disease that causes pain, swelling, stiffness, and loss of function in the joints. During rheumatoid arthritis, a subset of NK cells is expanded within the inflamed joints. It is believed that the NK cells then interact with the macrophage/monocyte population (i.e. myeloid cells) within the joint, thus amplifying the production of proinflammatory cytokines. By providing substances that block the interaction between NKp80 expressed on NK cells and AICL expressed on myeloid cells the release of proinflammatory cytokines could be strongly reduced, if not inhibited, in these conditions.

Besides of rheumatoid arthritis, all inflammatory rheumatic disorders could be treated and/or prevented by making use to the findings of the present invention. Inflammatory rheumatic disorders are caused by the same mechanisms as rheumatoid arthritis, i.e. by inflammatory reactions connected with the production of cytokines by NK cells that interact with myeloid cells.

In a preferred embodiment, the substance is selected from the group comprising anti-NKp80-antibodies, soluble NKp80, or functional fragments thereof.

With the mentioned substances, it is possible to selectively block the interaction between NKp80 and AICL, as shown by the inventors. The inventors generated and tested anti-NKp80-antibodies in view of their capability to block AICL/NKp80-interaction; further, the inventors tested soluble NKp80 (ectodomain-tetramers).

Antibodies according to the invention can be monoclonal or polyclonal, which can be easily obtained according to methods are known to a person skilled in the art. In particular monoclonal antibodies can be produced from hybridomas (see Kohler and Milstein, 256: 495 - 497, 1975).

As intended herein, segments of such antibodies, such as Fab, F(ab)'₂ or scFv fragments, and other fragments such as CDR ("complementarity-determining region", hypervariable region) fragments, are also considered as antibodies, as long as they show the same functionality as the antibodies. The said fragments exhibit the binding specificity of the antibody and can also be prepared recombinantly, for example using known methods.

For instance, NKp80-antibodies can be used which are commercially available from R & D Systems, Minneapolis, MN, USA

Further, in a preferred embodiment, the antibodies used in the present invention are humanized antibodies or fragments of humanized antibodies.

Humanized antibodies can be for example chimeric antibodies, in which, if appropriate, constant parts of animal antibodies, such as mouse or rabbit antibodies are replaced by the corresponding parts of human antibodies, such as the Fc fragment (Sharon et al., Nature 309: 364 - 367, 1984). Alternatively, the complex determining region (CDR) of the animal antibodies can be grafted onto human antibodies, a process called "Antibody Reshaping". Another alternative technique is to produce human antibodies in mice using transgenic animals.

In a preferred embodiment, an anti-NKp80 antibody is used which is produced by hybridoma cells that can be obtained by using soluble NKp80 ectodomain (NKp80-ED) for immunization of suitable animals, isolating anti-NKp80 antibody-producing lymphoid cells and subsequent fusion between a myeloma cell and the anti-NKp80 antibody-producing lymphoid cell.

In a preferred embodiment, an anti-NKp80 antibody is used, which is produced by hybridoma cells 5D12, which have been deposited at the Deutsche Sammlung für Mikroorganismen und Zellkulturen (DSMZ) under the acession number DSM ACC 2805.

The inventors of the present invention have generated anti-NKp80 monoclonal antibodies by immunizing mice with the NKp80-ectodomain (NKp80-ED). In order to demonstrate the specific interaction of AICL and NKp80 the inventors blocked AICL binding to NK cells by pre-treatment of NK cells with the NKp80 monoclonal antibodies, proofing suitability of said anti-NKp80-antibodies as substances within the context of the present invention.

The term "suitable animals" as it is used herein is intended to mean any animal which can be immunized with antigens and which subsequently produce antibody-producing lymphoid cells. In preferred embodiments the animals are mice or rabbits, and the lymphoid cells are spleen cells.

In addition to the above mentioned new anti-NKp80-antibodies, the inventors generated anti-AICL monoclonal antibodies by immunizing mice with the AICL-ectodomain (AICL-ED). In order to demonstrate the specific interaction of AICL and NKp80 the inventors blocked NKp80-ED tetramer binding by pre-treatment of AICL (either microsphere-immobilized or expressed at the surface of COS-7 cells) with the mentioned antibody 7F12.

In a further embodiment, a substance is used which comprises a amino acid sequence, which is selected of the group comprising SEQID No. 1, SEQ ID No. 2, of the enclosed sequence listing.

In yet another embodiment, a substance is used which consists of a amino acid sequence, which is selected of the group comprising SEQID No. 1, SEQ ID No. 2, SEQ ID No. 3 and SEQ ID No. 4 of the enclosed sequence listing.

The peptide listed in the enclosed sequence listing with SEQ ID No. 1 represents the full length amino acid sequence of NKp80 (231 AA). NKp80 displays a cytoplasmic domain (AA 1 to 38), a transmembrane domain (AA 39 to 68), and an ectodomain (AA 69 to 231). Amino acid sequence SEQ ID No. 2 of the enclosed sequence listing identifies the ectodomain of NKp80. SEQ ID No. 3 of he enclosed sequence listing identifies the full length amino acid sequence of AICL (AA 149), SEQ ID No. 4 the ectodomain of AICL (AA 26 to 149).

A person skilled in the art will recognize that also sequences related to the listed sequences are suitable in the context of the present invention, that comprise any kind of modification, e.g. amino acid exchanges, deletions or additions, but that still display the characteristic features, in particular binding properties, of the sequences listed herein. Also, such modified sequences can be used for immunizing suitable animals to subsequently generate hybridomas that produce antibodies directed against NKp80.

With the present use, it is preferred, if the substances, i.e. soluble NKp80 are recombinantly generated. In this connection, it can be suitable to express, e.g., the domain relevant for binding, and not the full length peptide/protein. For example, soluble AICL can be expressed without the cytoplasmic region and without the transmembrane domain. Insect cells like Sf9 or eukaryote cells like 293T or COS7 cells are suitable for expression of the mentioned peptide/fragments.

Thus, in an embodiment of the present invention soluble ectodomains (ED) of AICL or NKp80 are used, preferably tetramers of said NKp80-ectodomains.

The inventors demonstrated that AICL-ED tetramers bound to NKp80 and that NKp80-ED tetramers bound to AICL, whereby this binding could be inhibited by anti-NKp80-antibodies or anti-AICL-antibodies, respectively.

The present invention also relates to a pharmaceutical composition comprising as active substance a substance capable of blocking the interaction between NKp80 and AICL in association with a pharmaceutically acceptable carrier.

In a particularly preferred embodiment the present invention relates to an above-defined pharmaceutical composition comprising as active substance an anti-NKp80 antibody, in particular a humanized anti-NKp80 antibody, soluble NKp80, or functional fragments thereof, with or without the pharmaceutically acceptable carrier.

In an preferred embodiment, an anti-NKp80-antibody is used, which is selected from the group comprising 5D12, 10E4 and 12D11.

In a further embodiment, the use includes administering one or more substances blocking the interaction between NKp80 and AICL in combination with a second therapeutic agent, e.g. an therapeutic agent or agent for treating autoimmune diseases, inflammatory diseases or allergic reactions.

The subject to be treated can be a mammal, preferably a human.

In preferred embodiments, the substance can be administered to the subject systematically (e.g., orally, subcutaneously, intravenously, rectally, parentally, intramuscularly, interperitonally, transdermally, topically). In a preferred embodiment, the compound is administered on repeated basis, e.g., the compound can be administered two, four, six or more times a day, month or year. The administration can be repeated until improvement in the subject's condition is seen or expected.

In addition to the substance, which represents the active compound in the composition, this composition can according to another object also comprise suitable buffers, diluents or additives. Suitable buffers include, for example, Tris-HCl, glycine and phosphate, while suitable diluents include, for example, aqueous solutions of NaCl, lactose or mannitol. Suitable additives include, for example, detergents, solvents, antioxidants and preservatives. A review of the substances which can be used for compositions of this nature is given, for example, in: A. Kibbe, "Handbook of Pharmaceutical Excipients", 3rd Ed., 2000, American Pharmaceutical Association and Pharmaceutical Press.

Further, the invention relates to a method for modulating *in vitro* the activity of cells expressing NKp80 and/or AICL, characterized in that said cells are contacted respectively with AICL or fragments thereof, and/or with NKp80 or fragments thereof. In preferred embodiments, the NKp80 expressing cells are NK cells and the AICL expressing cells are myeloid cells, preferably malignant myeloid cells.

With the method disclosed it is possible to selectively affect the interaction between NKp80 and/or AICL expressing cells. Since the inventors of the present invention have identified AICL as a ligand of NKp80 it is possible to activate the NKp80 receptor by mediating binding of the ligand to its receptor and therefore to stimulate release of cytokines by the NK cells.

The inventors of the present invention have demonstrated that by contacting cells expressing AICL with freshly isolated NK cells said AICL expressing cells were lysed.

This cell lysis was mediated by the NK cells - most likely due to the NK cells' release of perforin and granzymes- and could be reduced by adding either anti-AICL monoclonal antibodies or soluble NKp80. In particular, the inventors were able to show strong lysis of malignant myeloid cells expressing high levels of AICL by isolated NK cells.

The inventors further demonstrated that by stimulating cells expressing AICL, AICL expression on these cells could be up-regulated upon exposure of said cells to ligands of Toll-like receptors (TLR), in particular with LPS, poly (Inosin:Cytosine), R848 or lipopeptide Pam₂Cys SK4. Monocytes, i.e. no-malignant myeloid cells, express AICL only at low levels and are resistant to NK lysis. However, when activating monocytes with LPS the inventors could demonstrate NK cytotoxicity against said monocytes, which could be inhibited by anti-NKp80/anti-AICl monoclonal antibodies. Thus, it was demonstrated that a TLR-mediated activation renders monocytes susceptible to NKp80 stimulated lysis by NK cells. Therefore, with the method according to the invention, an important tool is provided to modulate cytotoxicity of NK cells against AICL expressing cells.

Further, the invention relates to a method for screening substances suitable to be used for the treatment or prevention of autoimmune diseases, characterized in that the substances suitable to be used for the prevention or treatment of such diseases are selected on their property of blocking the interaction between NKp80 or fragments thereof and AICL or fragments thereof.

The terms "blocking the interaction between" means that substances are selected on their capability of inhibiting cytotoxicity of NKp80 expressing cells against AICL expressing cells, e.g. by binding to NKp80 or AICL and thus blocking the activating interaction between the two receptors.

Examples for substances being capable to act in the described way are antibodies, in particular anti-NKp80- or anti-AICL-antibodies, or fragments thereof, preferably monoclonal antibodies, and more preferably humanized antibodies.

With the findings presented by the inventors of the present invention it is possible to selectively block the interaction and therefore to inhibit NK cytotoxicity against AICL expressing cells, the fact of which can be utilized when treating or preventing autoimmune diseases.

It will be appreciated that the features mentioned above and yet to be explained hereinafter can be used not only in the combinations indicated in each case but also in other combinations or alone without leaving the scope of the present invention.

### DESCRIPTION OF PREFERRED EMBODIMENTS

### BRIEF DESCRIPTION OF THE FIGURES

Further advantages will be apparent from the following description of the examples and the attached figures. These show:

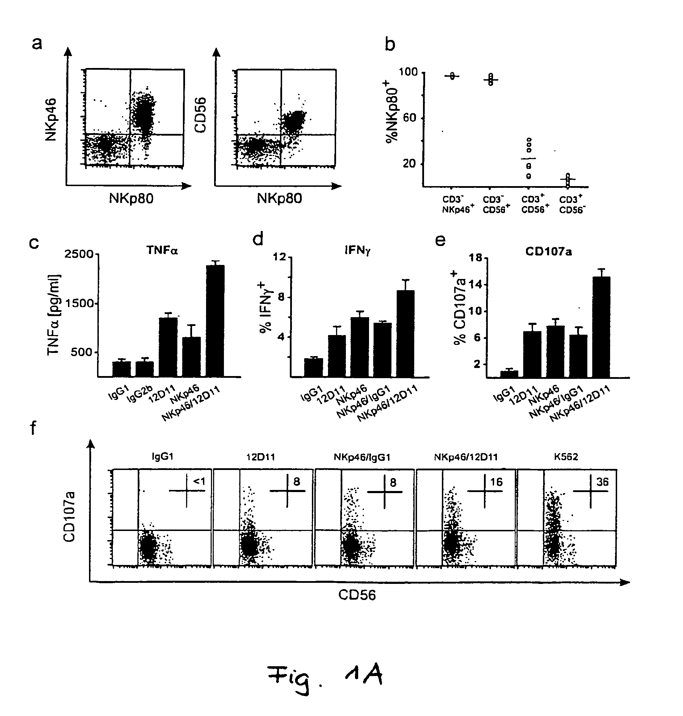

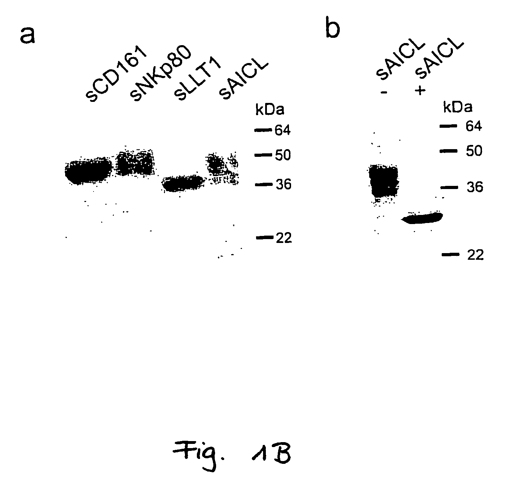

**Fig. 1A** **NKp80 stimulates granular exocytosis and cytokine secretion.**
   a) Human resting NK cells were analyzed for co-expression of NKp80 (mAb 5D12) and NKp46 (left panel), or CD56 (right panel), respectively. CD56^{bright} NK cells (gray) are also NKp80^{bright}. Plots show staining of freshly isolated PBMC where CD3-positive cells were excluded by electronic gating.
   (b) Frequencies of NKp80-positive cells among NK cells (CD3⁻, NKp46⁺ or CD3⁻, CD56⁺) and T cell subpopulations (CD3⁺, CD56⁺ or CD3⁻,CD56⁻) from 8 healthy donors. Resulting means of frequencies are indicated by horizontal bars.
   (c-f) Freshly purified NK cells were incubated with plate-bound anti-NKp80 mAb 12D11, anti-NKp46 mAb and appropriate isotype controls. (c) Concentrations of TNFα in culture supernatants were determined by ELISA and are shown as means of triplicates, error bars represent s.d. Results are representative of 3 independent experiments (d-f) Frequencies of CD107a⁺ and IFNγ⁺ NK cells were determined by flow cytometry. (d,e) Results are shown as means of triplicates with s.d. and are representative of 6 independent experiments. (f) Representative analysis of CD107a⁺ NK cells after stimulation with immobilized antibodies or K562 cells. Percentages of CD107a⁺ NK cells (upper right quadrant) are depicted.
**Fig.1B** **Recombinant soluble ectodomains (ED) of various C-type lectin-like receptors**
   (a) SDS-PAGE of soluble CD161-ED, NKp80-ED, LTT-ED, and AICL-ED affinity-purified from supernatants of transfected 293T cells. (b) SDS-PAGE of soluble AICL-ED untreated (-) or treated (+) with PNGase F.
**Fig.2A** **NKp80 ligates AICL.**
   (a) COS-7 cells 48h transiently transfected with FLAG-tagged full-length cDNA of AICL (gray histogram), LLT1 (hatched line), CD161 (thin black line), or the control vector (thick black line) were stained with anti-FLAG mAb M2.
   (b) Staining of microsphere-immobilized sAICL-ED (imAICL) (black histogram), imLLT1 (black line) or imNKp80 (dashed line) with sNKp80-ED tetramers (left panel) or sCD161-ED tetramers (right panel).
   (c) Staining of microsphere-immobilized sNKp80-ED (imNKp80) (black histogram), imCD161 (black line) or imAICL (dashed line) with sAICL-ED tetramers (left panel). Staining of imNKp80 (black histogram), imCD161 (black line) or imLLT1 (dashed line) with sLLT1 tetramers (right panel).
   (d) Blocking sAICL-ED tetramer binding to NKp80 by pre-incubation of imNKp80 with anti-NKp80 mAb 5D12 (hatched line) and 10E4 (dashed line), but not IgG1 isotype control (black histogram). AICL-ED tetramer staining of imAICL (black line) indicates background staining.
   (e) Freshly isolated human NK cells bind sAICL-ED tetramers and binding is blocked by NK cell pre-incubation with anti-NKp80 mAb 10E4 (gray histogram), but not with IgG1 control (black histogram). CD161-ED tetramer staining (black line) indicates background staining. NKp80 surface levels detected by mAb 5D12 (black histogram) and respective isotype control staining (black line) are depicted in the right panel.
   (f) Binding of sNKp80-ED tetramers to imAICL is partially blocked by anti-AICL mAb 7F12 (solid black line), but not by anti-AICL mAb 7G4 (hatched line) or an IgG1 control (black histogram). Staining of imNKp80 served as negative control (dotted line).
   (g) NKp80-ED tetramers bind COS-7 transiently transfected with an AICL-Ly49A-CD3ζ hybrid (second panel), and binding is impaired by pre-incubation of COS-7 with anti-AICL mAb 7F12 (third panel). NKp80-ED tetramers do not bind mock-transfected COS-7 (right panel). AICL-hybrid expression was monitored by anti-FLAG mAb M2 (left panel). Percentages of stained cells (upper left quadrant) are given.

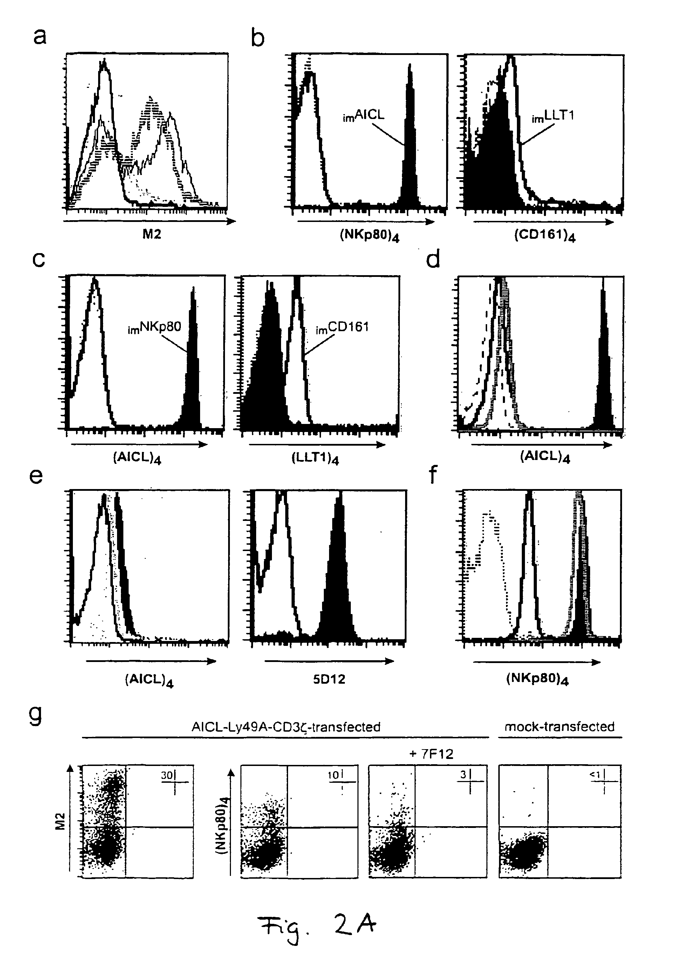

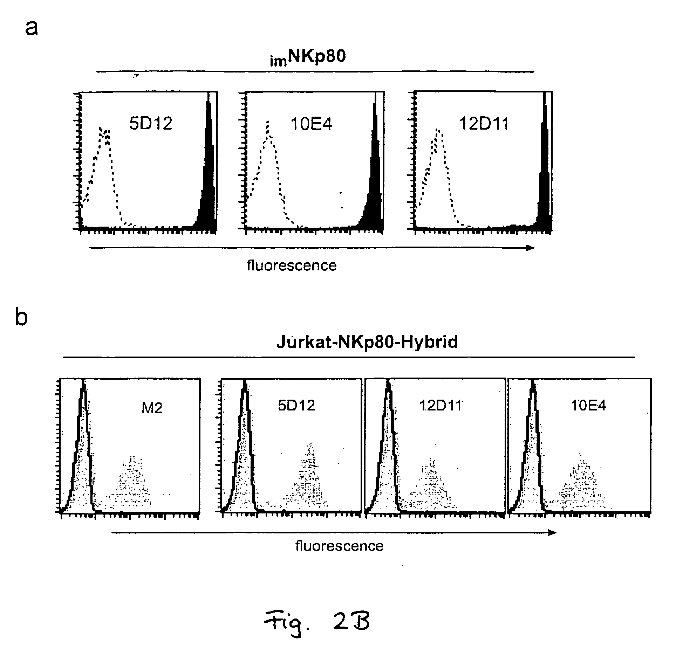

**Fig.2B** **Specificity of anti-NKp80 mAb 5D12, 10E4, and 12D11.**
   **(a)** 5D12, 10E4, and 12D11 bind to microsphere-immobilized NKp80 (imNKp80) (filled histograms), but not to imLTT1 (open histograms). (b) 5D12, 10E4, 12D11, and anti-FLAG mAb M2 show comparable staining patterns of a mixture of Jurkat cells trandsfected with the FLAG-tagged NKp80-CD69 hybrid cDNA and NKp80-hybrid-negative Jurkat transfectants (grey histograms). Open histograms represent the isotype control staining.

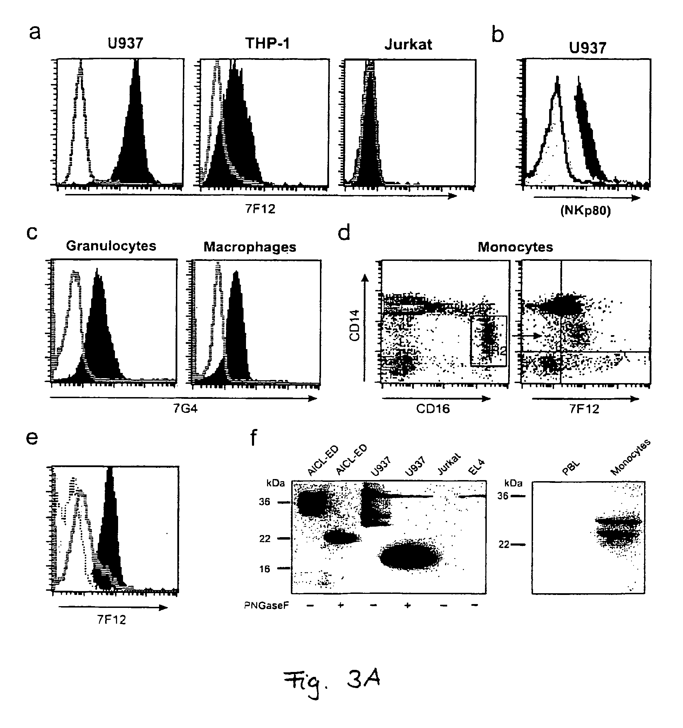

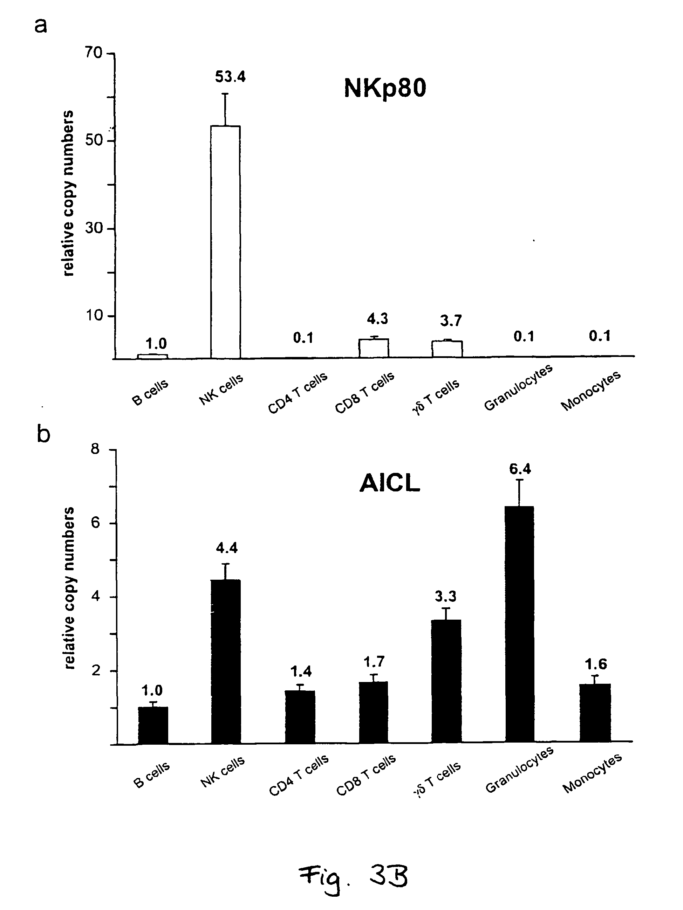

**Fig.3A** **AICL is a myeloid-specifc receptor.**
   (a) Anti-AICL mAb 7F12 stains myeloid cell lines U937 and THP-1, but not the T cell line Jurkat (filled histograms). IgG1 control stainings are open histograms.
   (b) NKp80-ED tetramers bind U937 cells and binding is impaired by pre-incubation of U937 cells with 7F12 (gray histogram), but not by an IgG1 control (black histogram). Staining with PE-conjugated streptavidin (SA) served as negative control (black line).
   (c) Freshly isolated granulocytes (CD66b⁺ cells) and in vitro matured macrophages (CD206⁺ cells) are stained by anti-AICL mAb 7G4. IgG1 control stainings are open histograms.
   (d) Freshly isolated CD14^{bright}CD16⁻ and CD14^{dim}CD16⁺ monocytes were stained with 7F12 (right panel) with CD14^{dim}CD16⁺ monocytes (gate R2) expressing higher AICL levels depicted in gray (right panel).
   (e) Monocytes down-regulate AICL during *in vitro* differentiation to immature DCs. Purified monocytes were cultivated with GM-CSF and IL-4 for 6 days and AICL expression detected by mAb 7F12 at day 0 (black histogram) and day 6 (hatched line) of culture. IgG1 control stainings at day 0 (dashed line) and day 6 (gray histogram) are overlayed. (a, c-e) All stainings were conducted after blocking Fc-receptors by pre-incubation with purified human Ig.
   (f) AICL in lysates of cell lines U937, Jurkat, and EL4 (left panel), freshly isolated monocytes and lymphocytes (PBL) (right panel) detected by immunoblotting with 7F12. Lysates were deglycosylated with PNGase F where indicated. Recombinant AICL-ED is included as positive control.
**Fig. 3B** **Abundance of AICL and NKp80 transcripts in leukocyte subpopulations**
   (a) Leukocytes from a healthy donor were FACS-sorted for CD3⁺CD8⁺ (CD8 T cells), CD3⁺CD4⁺ (CD4 T cells), CD3⁺γδ TCR+ (γδ T cells), CD19⁺ (B cells), CD66b⁺ (granulocytes), CD14⁺ (monocytes) and CD3-CD56⁺ (NK cells). ΔC_{τ} values for NKp80 (a) and AICL (b) transcripts were calculated by normalization with 18S RNA and relative copy numbers determined by setting the ΔC_{τ} value of B cells as 1.

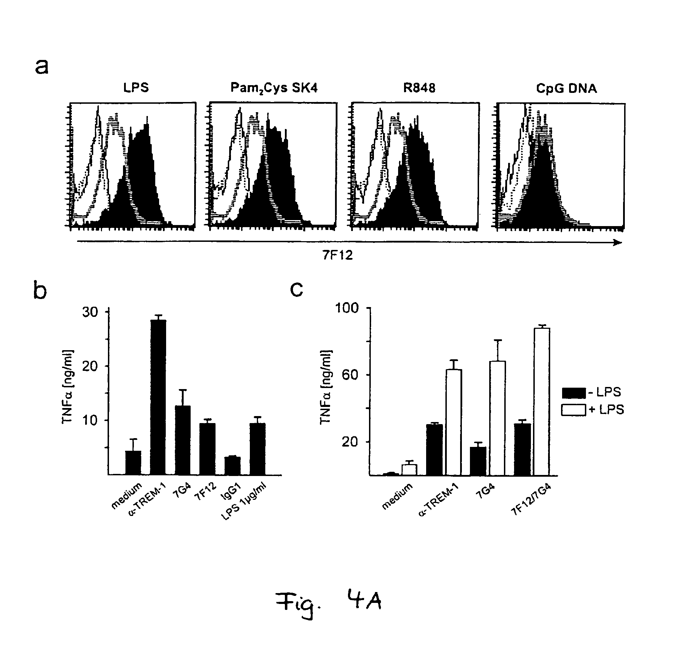

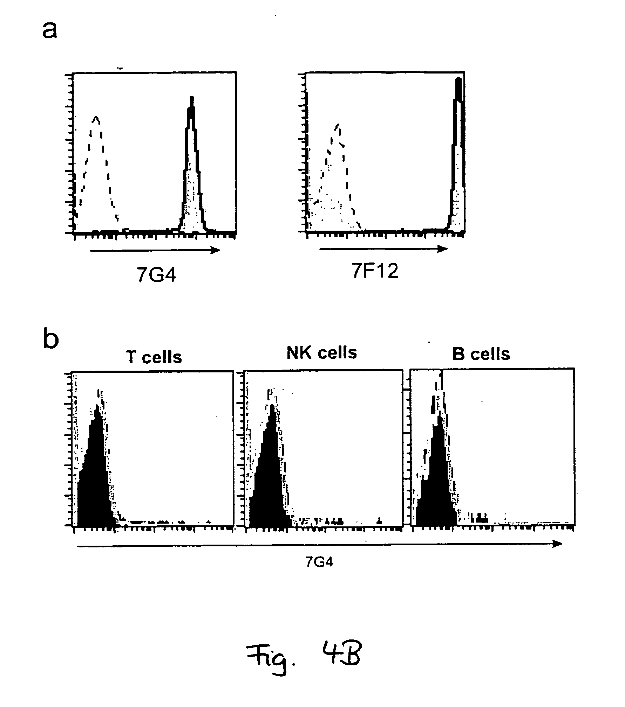

**Fig.4A** **AICL is up-regulated by TLR-ligands and stimulates TNFα release.**
   **(a)** Freshly purified monocytes stimulated for 24 h with 1 µg/ml LPS, 1 µM Pam₂Cys SK4, 10 µg/ml R848, or 10 µg/ml CpG 2216/2006, respectively, were stained with anti-AICL mAb 7F12 (black histograms) or an IgG1 control (dotted line). Stainings of mock-treated monocytes with 7F12 (hatched line) or an IgG1 control (black line) are overlayed.
   **(b)** TNFα-release by freshly isolated monocytes cultivated for 24h with plate-bound mAbs 7F12, 7G4, anti-TREM-1, IgG1 control, or LPS, respectively, was assayed by ELISA of culture supernatants.
   (c) TNFα-release by freshly isolated monocytes stimulated for 24h with plate-bound mAbs 7F12, 7G4, anti-TREM-1, in the presence (open bars) or absence of 1 µg/ml LPS (black bars). In b and c means of triplicates are shown, error bars represent s.d. All results are representative of at least 3 independent experiments.
**Fig.4B** **(a) Specificity of anti-AICL mAb 7F12 and 7G4.** 7F12 and 7G4 bind to microsphere-immobilized AICL ((imAICL) solid line), but not to imNKp80 (dashed line), and to about one third of a 2:1 mixture of imNKp80-microspheres and imAICL-microspheres (grey histograms). **(b) Lymphocytes** are bare of AICL. Freshly isolated T cells (CD3⁺), NK cells (CD56⁺) and B cells (CD19⁺) were not stained by anti-AICL mAb 7G4 (filled histogram). Open histograms represent isotype control stainings.

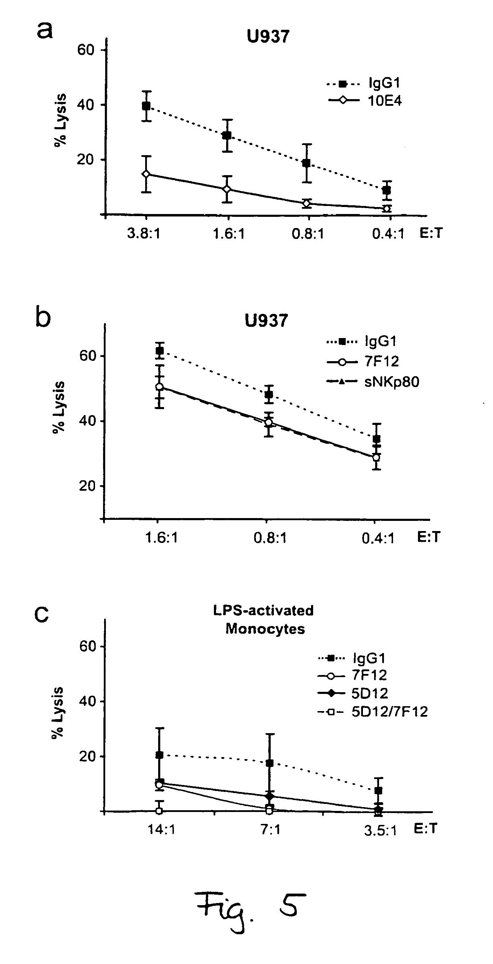

**Fig. 5****: NKp80-AICL interaction promotes NK lysis of myeloid cells.**
   **(a-c)** Cytotoxicity by freshly purified NK cells was determined in a 4h ⁵¹chromium-release assay
      **(a)** NK lysis of U937 cells in presence of anti-NKp80 mAb 10E4 (solid line) or a control IgG1 (dotted line) or
      **(b)** in presence of anti-AICL mAb 7F12 (dashed line), soluble NKp80-ED (solid line), or a control IgG1 (dotted line).
      **(c)** Lysis of LPS-activated, CD14-purified monocytes in presence of anti-NKp80 mAb 5D12 (solid line, filled diamonds), anti-AICL mAb 7F12 (solid line, open circles), a combination of 5D12 and 7F12 (solid line, open squares), or an IgG1 control (dotted line). F(ab')₂ fragments were used in all experiments. NK cells in (a-c) were from different donors with data as means of quadruplicates (a, b) or triplicates (c); errors bars represent s.d.

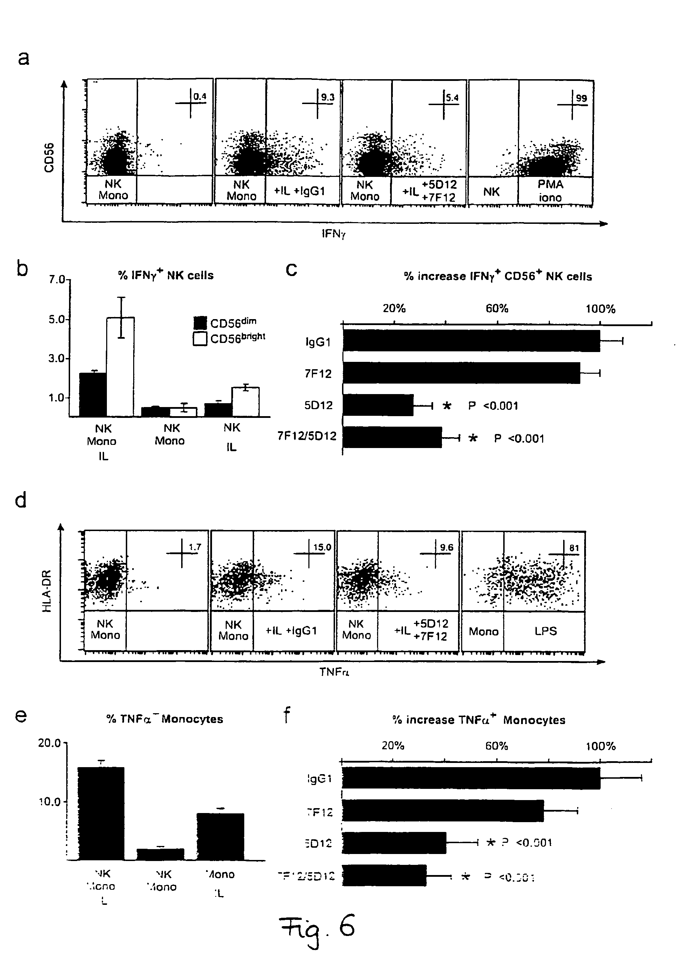

**Fig. 6****: NKp80-dependent stimulation of cytokine release by NK cells and monocytes.**
   **(a-c)** Frequencies of IFNγ-producing NK cells after 12h culture with autologous CD14^{dim}CD16⁺, monocytes. **(a)** Representative analysis of IFNγ⁺ NK cells cultured with monocytes (left panel), or with monocytes and monokines (IL) in the presence of both mAb 7F12 and 5D12 (third panel) or a control IgG1 (second panel). Stimulation with PMA and ionomycin served as positive control (right panel). Percentages of IFNγ⁺ CD56⁺ NK cells (upper right quadrant) are given.
   **(b)** Frequencies of IFNγ⁺ CD56^{dim} NK cells (black bars) or IFNγ⁺ CD56^{bright} NK cells (open bars) after culture with monocytes, monokines (IL) or both.
   (c) Increase in frequencies of IFNγ⁺ NK cells by co-culture with monocytes in the presence of monokines, mAb 7F12, 5D12, or both, or a control IgG1.
   (d-f) Frequencies of TNFα-producing CD14^{dim}CD16⁺ monocytes after 12 h culture with autologous NK cells. (d) Representative analysis of TNFα⁺ monocytes cultured with NK cells (left panel), or with NK cells and monokines (IL) in the presence of both mAb 7F12 and 5D12 (third panel) or a control IgG1 (second panel). LPS-stimulation served as positive control (right panel). Percentages of TNFα⁺ HLA-DR⁺ monocytes (upper right quadrant) are given. (e) Frequencies of TNFα⁺ monocytes after co-cultivation with NK cells, monokines (IL) or both.
   (f) Increase in frequencies of TNFα⁺ monocytes by co-culture with NK cells in the presence of monokines, and mAb 5D12, 7D12, or both, or a control IgG1. In c and f increase in presence of control IgG1 is set as 100% (for details see methods). All data are means of triplicates (a, donor 6) or quadruplicates (b-f, donor 3), errors bars represent s. d. p-values were calculated using the two-tailed Student's t test with alpha = 0,050: 1,000 and * indicates a significant difference in relation to the IgG1 isotype control. In all experiments mAbs were endotoxin-low F(ab')₂ -fragments.

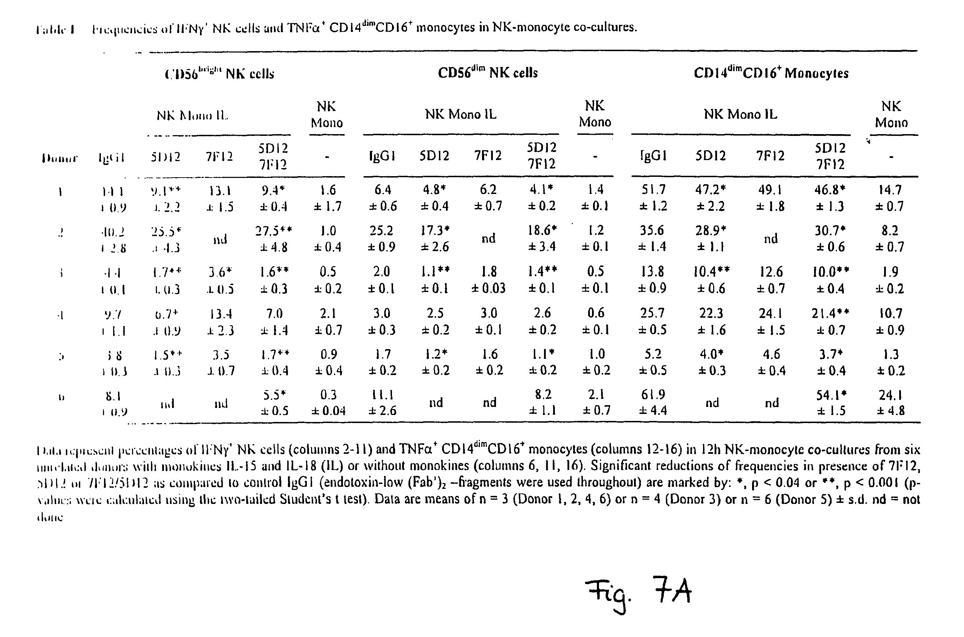

**Fig 7A****: Table 1: Frequencies of IFNγ⁺ NK cells and TNFα⁺ CD16⁺ monocytes in NK-monocytes co-cultures.**

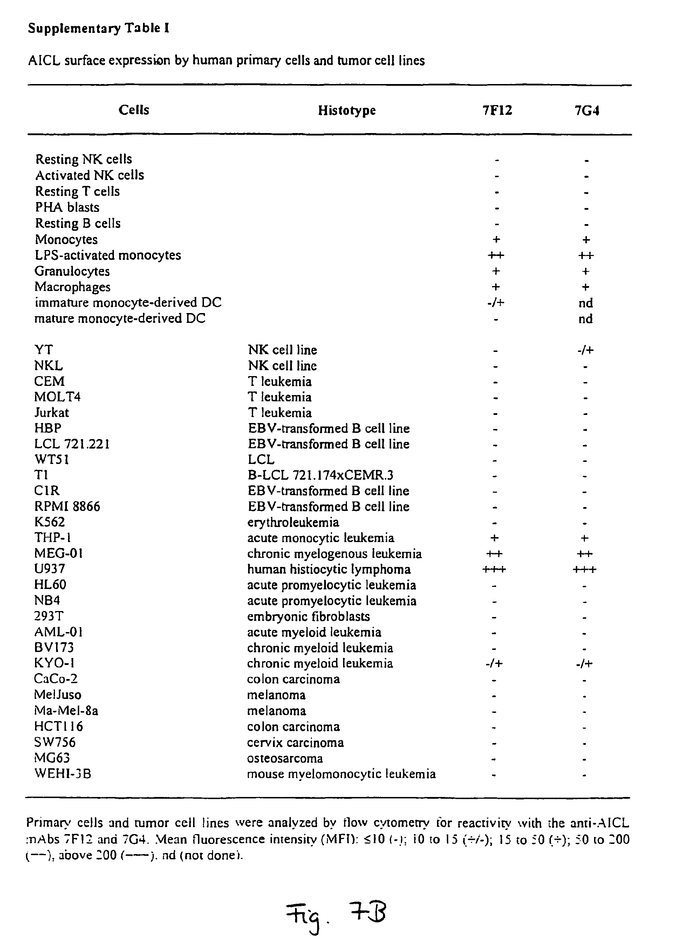

**Fig.7B****: Supplementary Table 1: AICL surface expression by human primary cells and tumor cell lines.**

### EXAMPLES

### Example 1: Methods

Cells. Peripheral leukocytes were obtained either from venous heparinized blood from healthy volunteers or from apheresis products obtained from the Center of Clinical Transfusion Medicine, Tübingen. NK cells were always purified by negative selection using the NK cell isolation kit II and CD16⁺ monocytes using the CD16⁺ Monocyte Isolation Kit (both from Miltenyi Biotec). For cytokine secretion assays, monocytes were negatively selected using the Monocyte Negative Isolation Kit (Miltenyi). For all other assays monocytes were purified by CD14 microbeads (Miltenyi). Cell purity was between 90-98% as assessed by flow cytometry. Granulocytes were isolated as described. Purified monocytes were differentiated to macrophages with 50 ng/ml hM-CSF. All cytokines were from R&D Systems except hIL-15 and hIL-2 (PromoCell). Freshly isolated cells were cultured in X-Vivo 15 (Cambrex) with 10% FCS (PAA). 293T cells and COS-7 cells were cultured in IMDM (Cambrex) supplemented with 10% FCS. Cell lines grown in suspensions were cultured in RMPI 1640 (Cambrex) with 10% FCS. All media were supplemented with penicillin (100 IU/ml)/streptomycin (100 µg/ml) (Cambrex), 2 mM L-glutamine (Cambrex) and 1 mM sodium pyruvate (c.c. pro). Surface AICL on purified monocytes was analyzed after a 24 h incubation with 1 µg/ml LPS from *Salmonella typhimurium* (Sigma), 1 µM S-[2,3-bis(palmitoyloxy)propyl]-cysteine-(Lys)₄ (Pam₂Cys SK4; EMC Microcollections), 50 µg/ml poly(I:C) (GE Healthcare), and 10 ng/ml R-848 (S. Bauer, Technical University of Munich, Munich, Germany).

Co-culture of NK cells and monocytes. NK cells and CD16⁺ monocytes were purified from apheresis products from healthy donors as described above and co-cultured at 4x10⁵ cells/well for 12 h at a 1:1 ratio (total 8x10⁵ cells/well) in complete X-Vivo medium containing 100 U IL-2/ml. In addition, IL-15 and IL-18 (both at 10 ng/ml) were added where indicated. For blocking of NKp80 and/or AICL, F(ab')₂ of 5D12 and 7F12, respectively, were added at 10 µg/ml. α-NP IgG₁ F(ab')₂ served as an isotype control. To assess frequencies of IFNγ-producing NK cells and TNFα-producing monocytes, CD56⁺ cells were stained for intracellular IFNγ, and CD14^{dim}16⁺HLA-DR^{bright} cells for intracellular TNFα, respectively, or with appropriate isotype controls. To calculate monocyte-dependent increase in frequencies of IFNγ-producing NK cells in the presence of blocking mAb, the monocyte-dependent increase in presence of an irrelevant IgG₁ (Fab')₂ was set as 100% ((%IFNγ-producing NK cells with monocytes) - (%IFNγ-producing NK cells without monocytes) = 100%). Increases in frequencies of TNFα⁺ monocytes by co-cultivation with NK cells was calculated accordingly.

Transfectants. Jurkat cells were transfected by electroporation with a NKp80-hybrid cDNA encoding the cytoplasmic and transmembrane domains of human CD69 (Met 1 through Gly 70), the NKp80 ectodomain (Gly 85 through Tyr 231), and a C-terminal FLAG-tag followed by a six-histidine-tag in RSV.5 neo. COS-7 cells were transiently transfected using FuGene6 (Roche) with an AICL hybrid cDNA encompassing the cytoplasmic domain of mouse CD3ζ (Arg 52 through Arg 164), the transmembrane domain of mouse Ly-49A (Ser 40 through Met 90), the AICL ectodomain (Lys 26 through His 149), and a C-terminal FLAG-tag followed by a six-histidine-tag in RSV.5 neo.

**NKp80- and AICL-specific monoclonal antibodies.** Splenocytes of mice repeatedly immunized with NKp80-ED or AICL-ED, respectively, were fused with P3X63Ag8.653 myeloma cells as described. Hybridoma supernatants were screened with mixtures of Jurkat-neo/Jurkat-NKp80 transfectants or mixtures of AICL-ED/LLT1-ED-coated microspheres by indirect immunofluorescence using a FACSCalibur (Becton Dickinson). Specificity was corroborated using COS-7 cells transiently transfected with NKp80 and AICL-Ly49A-CD3ζ hybrid cDNA, respectively (data not shown). Immunoglobulins of subcloned hybridoma were purified from supernatants with Protein A (Biorad) and isotyped by an ELISA isotyping kit (BD Biosciences). The mAb 5D12, 10E4 and 12D11 are NKp80-specific, the mAb 7F12 and 7G4 are AICL-specific, and all mAb are of IgG1 isotype. Antibodies were labelled using Alexa Fluor 647 carboxylic acid-succinimidyl ester according to the manufacturer's protocol (Molecular Probes).

Antibodies. Antibodies, if not stated otherwise, were purchased from BD Biosciences. PE-conjugated anti-NKp46, anti-NKp30 and anti-CD56 were supplied by Immunotech, CD14-FITC and isotype control from Immunotools, CD14-PE/Cy7 and isotype control from BioLegend. Unconjugated anti-NKp46 and anti-TREM-1 were from R&D Systems. Anti-FLAG-mAb M2 was from Sigma, anti-penta-His mAb from Qiagen, and goat anti-mouse-Ig-PE conjugate from Jackson Laboratories. The α-NP (NP = 4-hydroxy-3-nitrophenylacetyl) IgG₁ mAb (clone N1G9) hybridoma was kindly provided by Jörg Kirberg, Max-Planck-Institute of Immunobiology, Freiburg, and was used as isotype control for unlabelled mouse IgG1. For blocking in ⁵¹Cr-release assays or NK-monocyte co-culture experiments, (Fab')₂ fragments were generated by pepsin digestion followed by Protein A purification and purified from endotoxins by Triton 114 extraction. Concentration of endotoxins in mAb and (Fab')₂ preparations were tested using a *Limulus* amebocyte lysate assay (QCL-1000, Cambrex) and was below 0.1 EU/µg mAb after Triton 114 extraction.

**Cytotoxicity and degranulation assays, cytokine analysis.** Cytotoxicity was analyzed in a standard 4h ⁵¹chromium-release assay as described (Welte, S.A. et al. "Selective intracellular retention of virally induced NKG2D ligands by the human cytomegalovirus UL16 glycoprotein." Eur. J. Immunol. 33, 194-203 (2003)). Degranulating NK cells were quantified by flow cytometry of surface CD107a after 6 h incubation with plate-bound mAb in the presence of 10 µg/ml Brefeldin A (Sigma) as described (Alter, G., . et al, "CD107a as a functional marker for the identification of natural killer cell activity." J. Immunol. Methods 294, 15-22 (2004). Likewise, frequencies of cytokine-producing NK cells were determined by intracellular staining with anti-IFNγ-PE after 6 h incubation with plate-bound mAb in the presence of 10 µg/ml Brefeldin A and 100 U IL-2/ml. As positive control ionomycin (Sigma) and PMA (Cell Signaling Technology) were used at concentrations of 1 nM and 10 ng/ml, respectively. TNFα concentrations in supernatants of purified NK cells stimulated for 24 h with plate-bound mAb and 100 U IL-2/ml were determined using ELISA CytoSets from BioSource. Likewise, TNFα in supernatants of purified monocytes was assayed after 24 h stimulation with plate-bound, endotoxin-low mAb.

**Soluble ectodomains (ED) of C-type lectin-like receptors.** Ectodomains of NKp80 (Gln 64 through Tyr 231), AICL (Lys 26 through His 149), LLT1 (Ala 61 through Val 191), and CD161 (Ile 66 trough Ser 225) were expressed in 293T cells stably transfected with the corresponding cDNA containing an N-terminal BirA-tag as well as C-terminal c-myc- and six-histidine-tags in pSecTag2/HygroC (Invitrogen). Stable transfectants were selected with 0.2 mg/ml Hygromycin B (Roche). Purified ED were prepared from supernatants of 293T-transfectants by affinity chromatography with anti-c-myc-mAb columns (clone 9E10, ATCC CRL-1729). Purified ED were biotinylated using *E.coli* expressed BirA Ligase as decribed (Welte. *et al., supra*)*.* Biotinylated ED were purified by size exclusion chromatography, analyzed by SDS-PAGE (Fig. 1B), and biotinylation verified by a gel shift assay with streptavidin (data not shown). Before use, biotinylated ED were either immobilized on streptavidin-coated microspheres (Bangs Laboratories) or tetramerized using phycoerythrin (PE)- or allophycocyanin (APC)-labelled streptavidin (Molecular Probes).

**Immunoblot analysis.** Immunoblotting was performed essentially as previously described (Waldhauer, I. & Steinle, A. "Proteolytic release of soluble UL16-binding protein 2 from tumor cells." Cancer Res. 66, 2520-2526 (2006)). Treatment with Peptide:N-Glycanase F (PNGaseF) (New England Biolabs) was for 1 h at 37 °C. Samples were blotted onto Hybond-ECL membranes (GE Healthcare), analyzed with 30 µg 7F12/ml, and detected with a goat anti-mouse HRP-conjugate (Jackson Laboratories).

**Real-time RT-PCR.** Total RNA was prepared using TRIZOL (Invitrogen) and reverse transcribed by SuperScript II (Invitrogen). The cDNA was amplified with NKp80, AICL and 18S rRNA specific primer pairs in duplicates (40 cycles, 95°C x 15 s, 60°C x 1 min) using SYBRGreen chemistry on the ABI PRISM 7000 Sequence Detection System (Applied Biosystems). Primers were selected to flank an intron, where possible, and specificity was validated using cloned cDNA. Data analysis was by the C_{τ} method for relative quantification. Similar amplification efficiencies for NKp80, AICL and 18S were demonstrated by analyzing serial cDNA dilutions with values of the slope of log cDNA amount vs. C_{τ} of < 0.1. Oligonucleotide sequences (forward; reverse) were for 18S rRNA: 5'-CGGCTACCACATCCAAGGAA-3'; 5'-GCTGGAATTACCGCGGCT-3'; NKp80: 5'- TTCAGTGACGTTGCACTGGT-3'; 5'-CTCCCTGAGAAACCAACAGGA-3'; AICL: 5'-TACCAAATCGTTTGGCATGA-3'; 5'-CTGCAAATCCATTTTCTTTCG-3'. PCR products were analyzed on 3% agarose gels for purity.

### Example 2: NKp80 stimulates degranulation and cytokine release of NK cells

In order to pursue extensive analyses of NKp80 expression and function, a panel of anti-NKp80 monoclonal antibodies was generated by immunizing mice with the NKp80-ectodomain (NKp80-ED). The tagged NKp80-ED was expressed in 293T cells and purified from the supernatants by affinity chromatography (Fig. 1B). Specificity of the resulting anti-NKp80 mAbs 5D12, 10E4, and 12D11 was verified in binding analyses using microsphere-immobilized NKp80-ED and NKp80-transfected Jurkat cells (Fig. 2B). In accord with previous reports, the anti-NKp80 mAb bound to nearly all freshly isolated human NK cells (Vitale, M. et al. "Identification of NKp80, a novel triggering molecule expressed by human NK cells". Eur. J. Immunol. 31, 233-242 (2001)) (Fig. 1A-a). It was also noted that the CD56^{bright} NK subset, which is a primary source of NK cytokines in response to monokines (Cooper, M.A. et al. "Human natural killer cells: a unique innate immunoregulatory role for the CD56(bright) subset." Blood 97, 3146-3151 (2001)), also brightly expresses NKp80. For some donors, NKp80 expression has also been reported for CD3⁺CD56⁺ cells. Accordingly, in the present studies, NKp80 was detected on varying fractions of CD56⁺CD3⁺ cells and even few CD56⁻CD3⁺ T cells depending on the individual donor (Fig. 1A-b). In contrast, B cells, monocytes and all tested cell lines were devoid of NKp80 (data not shown). These findings were supported by real-time RT-PCR analyses showing a high abundance of NKp80 transcripts in NK cells (Fig. 3B).

The impact of NKp80 triggering on cytokine release by NK cells has not yet been addressed. To investigate the consequences of NKp80 triggering for NK effector functions independently of other NK receptors, freshly purified NK cells were incubated with plate-bound anti-NKp80 mAb. Cross-linking of NKp80 induced marked secretion of TNFα by NK cells (Fig. 1A-c). Simultaneous stimulation by anti-NKp80 and anti-NKp46 resulted in an about 2-fold increase in secretion of TNFα suggesting that NKp46 and NKp80 act synergistically. Similar results were obtained when frequencies of IFNγ-secreting NK cells were determined upon triggering of NKp80 and/or NKp46 (Fig. 1A-d), demonstrating that NKp80 is capable to stimulate NK cytokine secretion. In accord with published data on NKp80-mediated stimulation of NK cytotoxicity (Vitale, M. et al. "Identification of NKp80, a novel triggering molecule expressed by human NK cells". Eur. J. Immunol. 31, 233-242 (2001)), it was observed that immobilized anti-NKp80 mAb also induced NK cell degranulation similarly to anti-NKp46, again with a pronounced cooperative effect of both receptors (Fig. 1A-e, f).

### Example 3: NKp80 engages the adjacently encoded C-type lektin-like receptor AICL

Further elucidation of the immunological relevance of NKp80-mediated NK activation necessitated the identification of NKp80-ligands (NKp80-L). It was attempted to define NKp80-L bearing cells implementing BWZ.36 cells expressing NKp80-CD3ζ reporter constructs, since ligands of the NKC-encoded mouse Nkrp1 receptors were previously identified using BWZ.36 cells expressing Nkrp1-CD3ζ reporter construct (Carlyle, J.R. et al. "Missing self-recognition of Ocil/Clr-b by inhibitory NKR-P1 natural killer cell receptors." Proc. Natl. Acad. Sci. U. S. A 101, 3527-3532 (2004) and lizuka, K., et al. "Genetically linked C-type lectin-related ligands for the NKRP1 family of natural killer cell receptors." Nat. Immunol. 4, 801-807 (2003)). These reports had revealed that mouse C-type lectin-like Nkrp1 receptors and their ligands, the so-called C-type lectin-related (Clr) molecules, both are encoded in close genetic linkage in the NKC. Since this strategy failed to identify NKp80-L expressing cells, the possibility was considered that the orphan genes Lectin-Like Transcript 1 (LLT1) and Activation-Induced C-type Lectin (AICL) encoded in close linkage to NKp80 in the human NKC might be ligands of NKp80 analogous to mouse Nkrp1-Clr pairs. In fact, during this work was in progress, LLT1 was reported to constitute a ligand of the single human representative of the Nkrp1 receptor family, NKRP1A/CD16, which is also genetically linked. In contrast to CD161, there are no known mouse homologues neither for NKp80 nor AICL. FLAG-tagged AICL was hardly detectable at the surface of COS-7 or 293T cells upon transient transfection as opposed to CD161 and LLT1 (Fig. 2A-a and data not shown). Hence, to directly assay a possible interaction of NKp80 with AICL or LLT1, soluble ectodomains of AICL (AICL-ED) and LLT1 (LLT1-ED) were produced using stably transfected 293T cells (Fig. 1B). AICL-ED or LLT1-ED, respectively, were immobilized on streptavidin-coated microspheres and directly tested for binding by fluorochrome-labelled NKp80-ED or CD161-ED tetramers, respectively, in flow cytometry. As expected, CD161-ED-tetramers bound immobilized LLT1 though staining was fairly weak indicating a low affinity interaction in agreement with recent reports (Aldemir, H. et al. "Cutting edge: lectin-like transcript 1 is a ligand for the CD161 receptor." J. Immunol. 175, 7791-7795 (2005) and Rosen, D.B. et al. "Cutting edge: lectin-like transcript-1 is a ligand for the inhibitory human NKR-P1A receptor." J. Immunol. 175, 7796-7799 (2005)).

(Fig. 2A-b). In contrast, NKp80-ED-tetramers did not bind to LLT1, but exhibited strong binding to immobilized AICL (Fig. 2A-b). Accordant results were obtained in a reversed setting with immobilized NKp80-ED specifically interacting with AICL-ED-tetramers (Fig. 2A-c). Further, AICL-ED-tetramer binding was blocked by pre-incubation of NKp80-ED-coated microspheres with various anti-NKp80 mAb (Fig. 2A-d). Importantly, AICL-ED-tetramers also stained freshly isolated NK cells and binding was blocked by pre-treatment of NK cells with NKp80 mAb demonstrating that AICL is a natural ligand of NKp80 (Fig. 2A-e).

### Example 4: Novel AICL-specific antibodies

There is only a single study on AICL reporting a differential AICL mRNA expression for T and B lymphocytes, monocytes and granulocytes (Hamann, J., et al. "AICL: a new activation-induced antigen encoded by the human NK gene complex." Immunogenetics 45, 295-300 (1997)). By real-time PCR, it could be confirmed that AICL transcripts are most abundantly expressed by granulocytes, and found these more prominently in NK cells and γδ T cells than in αβ T cells or B cells (Fig. 3B). However, AICL protein expression is unknown due to the lack of appropriate antibodies. Thus, to explore AICL expression, AICL-specific mAb were generated by immunizing mice with AICL-ED. Two AICL-specific mAb, 7F12 and 7G4, were obtained that bound immobilized AICL-ED, but not LLT1-ED, NKp80-ED or CD161-ED, and also stained COS-7 cells transiently transfected with AICL-Ly49A-CD3ζ hybrids where transmembrane and cytoplasmic sequences of AICL were replaced by mouse Ly49A (transmembrane) and mouse CD3ζ (cytoplasmic) sequences (Fig. 4B and data not shown). Pre-incubation of microsphere-immobilized AICL with 7F12, but not with 7G4, reduced binding of NKp80-ED tetramers indicating that 7F12 partially hinders NKp80-AICL interaction (Fig. 2A-f). Importantly, NKp80-ED tetramers also bound the AICL ectodomain expressed at the surface of COS-7 cells, and addition of 7F12 interfered with binding (Fig. 2A-g).

### Example 5: AICL is a myeloid-specific surface receptor

Next, AICL surface expression was analysed by various cell lines and detected AICL on myeloid cell lines U937, THP-1 and MEG-01 (Fig. 3A-a and Supplementary Table Fig. 7B). U937 cells, most prominently expressing AICL, also strongly stained with NKp80-ED tetramers, and pre-incubating U937 with anti-AICL 7F12 markedly reduced NKp80-ED binding (Fig. 3A-b). In contrast to myeloid cell lines, AICL was not detectable on non-myeloid hematopoietic or on non-hematopoietic cell lines (Fig. 3A-a and Supplementary Table Fig. 7B) suggesting that AICL is preferentially expressed at the surface of myeloid cells. Thus, AICL expression by peripheral blood leukocytes was analysed and observed specific binding of 7F12 and 7G4 to monocytes, macrophages and granulocytes, but not to T cells, B cells, or NK cells (Fig. 3A-c,d and Fig. 4B). Among monocytes, the CD14^{dim}CD16⁺ subset which is a major source of TNFα exhibits substantially higher AICL surface levels than the CD14^{bright}CD16⁻ subset (Fig. 3A-d). Also, AICL expression by DCs was addressed, because the cellular cross-talk between NK cells and DCs has attained much interest. Interestingly, AICL was strongly down-regulated when monocytes were differentiated in vitro to immature DCs (Fig. 3A-e) indicating that NKp80-AICL interaction may not be involved in the interaction of NK cells with monocyte-derived DCs. A previous report suggested that NKp80-L may be expressed by activated T cells, since NK cytotoxicity against PHA-activated T cells was partially reduced by addition of anti-NKp80 mAb (Vitale *et al., supra*). However, AICL could not be detected at the surface of activated T cells (Supplementary Table Fig. 7B). Myeloid-specific AICL expression was surprising given that a previous report (Hamann *et al., supra)* and the present analyses detected AICL transcripts also in lymphocytes. Therefore, AICL protein was analysed in whole cell lysates using anti-AICL mAb 7F12. In accordance with AICL surface expression by U937 cells and monocytes, AICL was detected in the respective cell lysates, but not in lysates of non-myeloid cell lines or lymphocytes (Fig. 3A-f). Altogether these data define AICL as myeloid-specific surface receptor.

### Example 6: AICL triggers TNFα-release by monocytes in synergy with LPS

Ligands of Toll-like receptors (TLR) are known to modulate cell surface expression levels of various immunoreceptors, including TREM-1, CD80 and CD83 (Bouchon, et al., "Cutting edge: inflammatory responses can be triggered by TREM-1, a novel receptor expressed on neutrophils and monocytes." J. Immunol. 164, 4991-4995 (2000)). Hence, modulation of AICL expression by TLR ligands was analyzed. In fact, AICL was markedly up-regulated upon exposure of monocytes to LPS, poly (I:C), R848, or Pam₂Cys SK4 within 24 hours, whereas CpG DNA had no effect as expected (Fig. 4A-a and data not shown). Next, stimulatory capacities of AICL were assessed. Therefore, anti-AICL mAb were immobilized and incubated for 24h with freshly isolated monocytes. Like LPS-treatment or stimulation with anti-TREM-1 mAb, AICL cross-linking resulted in a markedly enhanced release of TNFα by monocytes (Fig. 4A-b). In addition, LPS exerted a strong synergistic effect on AICL-stimulated TNFα release (Fig. 4A-c).

### Example 6: NKp80 promotes lysis of AICL-expressing malignant myeloid cells

Previous studies demonstrated that NKp80 stimulates NK cytotoxicity in redirected lysis assays when cross-linked by anti-NKp80 mAb (Vitale *et al.,* supra). However, due to the unknown nature of NKp80-L, relevance of NKp80-dependent cytotoxicity in a biological relevant setting could not be assessed. Here, the impact of NKp80 for NK cytotoxicity towards myeloid cells expressing AICL was addressed. U937 cells express high levels of AICL, but also of ligands of the activating NK receptor DNAM-1. Accordingly, freshly isolated NK cells strongly lysed U937. U937 lysis was partially blocked by anti-NKp80 mAb 10E4 demonstrating that NKp80 markedly contributes to U937 lysis (Fig. 5A-a). Further, addition of either anti-AICL mAb 7F12 or soluble NKp80 also reduced NK cytotoxicity against U937 (Fig. 5A-b). In contrast to U937, non-malignant myeloid cells like monocytes only express low levels of AICL and DNAM-1 ligands and are largely resistant to NK lysis (data not shown). However, upon activating monocytes by LPS for 24h, in case of some donors moderate cytotoxicity by autologous NK cells was observed that was inhibited by anti-NKp80/anti-AICL antibodies (Fig. 5A-c and data not shown) indicating that TLR-mediated activation may render monocytes susceptible to NKp80-stimulated lysis by NK cells.

### Example 7: NKp80-dependent mutual activation of NK cells and monocytes

In a recent report (Dalbeth, N. et al. "CD56bright NK cells are enriched at inflammatory sites and can engage with monocytes in a reciprocal program of activation." J. Immunol. 173, 6418-6426 (2004)), a bi-directional activation pathway between NK cells and monocytes was described resulting in secretion of IFNγ and TNFα by NK cells and monocytes, respectively. It was suggested that this mutual activation may occur at sites of inflammation, particular of chronic inflammatory autoimmune diseases where activated CD56^{bright} NK cells and monocytes are prominent. It was shown that co-culture of NK cells and monocytes in presence of monokines resulted in a markedly increased secretion of pro-inflammatory cytokines by both cell types that was partially cell contact-dependent. However, the receptors accounting for the cell contact-dependent activation remained unknown. In the present studies, this experimental system was adopted and confirmed that co-culture of freshly isolated, autologous NK cells and monocytes markedly increased the frequencies of IFNγ-secreting NK cells and TNFα-secreting monocytes, respectively, as compared to single cultures of NK cells and monocytes (Fig. 6). In accord with previous studies, CD56^{bright} NK cells were more prone to produce IFNγ than CD56^{dim} NK cells (Fig. 6b, and Table I Fig. 7A). Importantly, addition of monokines IL-15 and IL-18 was essential to induce cytokine secretion.

To investigate whether NKp80-AICL interaction may account for the reported cell contact-dependency of the activating cross-talk, F(ab')₂ fragments of anti-NKp80 mAb 5D12 and/or anti-AICL mAb 7F12 were added to NK-monocyte co-cultures. Importantly, blocking NKp80 strongly reduced the monocyte-dependent increase of IFNγ secretion by NK cells demonstrating that NKp80 is crucially involved in the activating NK-monocyte crosstalk (Fig. 6a, c). Though the frequencies of IFNγ-secreting CD56^{bright} NK cells varied widely between various donors (range 3.8% to 40.2%), NKp80 blockade always resulted in a strong reduction of responsive cells (Table I Fig. 7A). Similarly, frequencies of IFNγ-secreting CD56^{dim} NK cells (range 1.7% to 25.2 %) were markedly reduced in four out of five donors analyzed. In contrast, mAb 7F12 did not significantly affect IFNγ-secretion by NK cells presumably due to its inefficient blocking capacities. Conversely, enhanced TNFα-secretion by monocytes through co-culture with NK cells ranged between 5% and 62%. Among four out of five donors TNFα-secretion was notedly reduced when NKp80 was blocked (Fig. 6d,f and Table I Fig. 7A), demonstrating that NKp80 engagement also contributes to the cell contact-dependent TNFα-secretion by monocytes.

## Claims

1. A substance blocking the interaction between NKp80 and its ligands on myeloid cells for use in the prevention or treatment of inflammatory diseases, **characterized in that** the substance is selected from the group comprising anti-NKp80-antibodies, soluble NKp80, or functional fragments thereof.

2. The substance according to claim 1, wherein the ligand is AICL.

3. The substance according to claim 1 or 2, wherein the inflammatory disease is an autoimmune disorder.

4. The substance according to any of claims 1 to 3, wherein the inflammatory disorder is an inflammatory rheumatoid disorder, in particular rheumatoid arthritis or a rheumatoid arthritis related condition.

5. The substance of any of claims 1 to 4, wherein a substance is used which comprises an amino acid sequence, which is selected of the group comprising SEQ ID No. 1 and SEQ ID No. 2 of the enclosed sequence listing.

6. The substance of any of claim 1 to 5, wherein the antibodies are humanized antibodies or fragments of humanized antibodies.

7. The substance of any of claims 1 to 5, wherein an anti-NKp80 antibody is used which is produced by hybridoma cells, that can be obtained by using soluble NKp80 ectodomain (NKp80-ED) for immunization of suitable animals, isolating anti-NKp80 antibody-producing lymphoid cells and subsequent fusion between a myeloma cell and the anti-NKp80 antibody-producing lymphoid cell.

8. The substance of any of claims 1 to 7, wherein an anti-NKp80 antibody is used which is produced by hybridoma cells 5D12.

9. The substance of any of claims 1 to 5, wherein soluble ectodomain of NKp80 are used.

10. Pharmaceutical composition comprising as active substance a substance capable of blocking the interaction between NKp80 and its ligand on myeloid cells, in association with an pharmaceutically acceptable carrier, wherein the substance is selected from the group comprising an anti-NKp80-antibody, which is produced by hybridoma cells 5D12, soluble NKp80, or functional fragments thereof.

11. Pharmaceutical composition according to claim 10, comprising as active substance a humanized anti-NKp80-antibody.

12. Method for modulating in *vitro* the activity of cells expressing NKp80 and/or AICL, **characterized in that** said cells are contacted respectively with AICL or fragments thereof, and/or with NKp80 or fragments thereof.

13. Method of claim 12, wherein the NKp80 expressing cells are NK cells.

14. Method of claim 12 or 13, wherein the AICL expressing cells are myeloid cells, in particular malignant myeloid cells.

15. A method for screening compounds suitable to be used for the treatment or prevention of autoimmune diseases, **characterized in that** the substances suitable to be used for the prevention or treatment of such diseases are selected on their property of blocking the interaction between NKp80 or fragments thereof and AICL or fragments thereof.

## Patentansprüche

1. Substanz zur Blockierung der Wechselwirkung zwischen NKp80 und dessen Liganden auf myeloide Zellen zur Verwendung in der Prävention oder Behandlung entzündlicher Krankheiten, **dadurch gekennzeichnet, dass** die Substanz ausgewählt ist aus der Gruppe umfassend Anti-NKp80-Antikörper, lösliches NKp80, oder funktionelle Fragmente davon.

2. Substanz nach Anspruch 1, wobei der Ligand AICL ist.

3. Substanz nach Anspruch 1 oder 2, wobei die entzündliche Erkrankung eine Auto-immunerkrankung ist.

4. Substanz nach einem der Ansprüche 1 bis 3, wobei die entzündliche Erkrankung eine entzündliche rheumatoide Erkrankung ist, insbesondere Rheumatoidarthritis oder ein mit Rheumatoidarthritis in Verbindung stehendes Leiden.

5. Substanz nach einem der Ansprüche 1 bis 4, wobei eine Substanz eingesetzt wird, die eine Aminosäurensequenz aufweist, die ausgewählt ist aus der Gruppe umfassend SEQ ID Nr. 1 und SEQ ID Nr. 2 des beigefügten Sequenzprotokolls.

6. Substanz nach einem der Ansprüche 1 bis 5, wobei die Antikörper humanisierte Antikörper oder Fragmente humanisierter Antikörper sind.

7. Substanz nach einem der Ansprüche 1 bis 5, wobei ein Anti-NKp80-Antikörper eingesetzt wird, der von Hybridomzellen produziert wird, die erhalten werden, indem die lösliche NKp80-Ektodomäne (NKp80-ED) zur Immunisierung geeigneter Tiere eingesetzt wird, Anti-NKp80-Antikörper-produzierende lymphoide Zellen isoliert werden und anschließende eine Myelomzelle und die Anti-NKp80-Antikörper-produzierende lymphoide Zelle fusioniert werden.

8. Substanz nach einem der Ansprüche 1 bis 7, wobei ein NKp80-Antikörper eingesetzt wird, der durch die Hybridomzelllinie 5D12 produziert wird.

9. Substanz nach einem der Ansprüche 1 bis 5, wobei die lösliche Ektodomäne von NKp80 eingesetzt wird.

10. Pharmazeutische Zusammensetzung, welche als Wirkstoff eine Substanz aufweist, die dazu in der Lage ist, die Wechselwirkung zwischen NKp80 und dessen Liganden auf myeloiden Zellen zu blockieren, in Verbindung mit einem pharmazeutisch akzeptierbaren Träger, wobei die Substanz ausgewählt ist aus der Gruppe umfassend einen Anti-NKp80-Antikörper, der von den Hybridomzellen 5D12 produziert wird, lösliches NKp80, oder funktionelle Fragmente davon.

11. Pharmazeutische Zusammensetzung nach Anspruch 10, welche als Wirkstoff einen humanisierten Anti-NKp80-Antikörper aufweist.

12. Verfahren zur invitro-Modulierung der Aktivität von Zellen, die NKp80 und/oder AICL exprimieren, **dadurch gekennzeichnet, dass** die Zellen jeweils mit AICL oder Fragmenten davon, und/oder mit NKp80 oder Fragmenten davon in Kontakt gebracht werden.

13. Verfahren nach Anspruch 12, wobei die NKp80-exprimierenden Zellen NK-Zellen sind.

14. Verfahren nach Anspruch 12 oder 13, wobei die AICL-exprimierenden Zellen myeloide Zellen, insbesondere maligne myeloide Zellen sind.

15. Verfahren zum Screenen von Verbindungen, die dazu geeignet sind, für die Behandlung oder Prävention einer Immunerkrankung eingesetzt zu werden, **dadurch gekennzeichnet, dass** die Substanzen, die für die Prävention oder die Behandlung solcher Krankheiten eingesetzt werden können, hinsichtlich ihrer Eigenschaft ausgewählt werden, die Wechselwirkung zwischen NKp80 oder Fragmenten davon und AICL oder Fragmenten davon zu blockieren.

## Revendications

1. Substance bloquant l'interaction entre NKp80 et ses ligands sur des cellules myéloïdes pour utilisation dans la prévention ou le traitement de maladies inflammatoires, **caractérisé en ce que** la substance est choisie dans le groupe comprenant des anticorps anti-NKp80, NKp80 soluble, ou des fragments fonctionnels de ceux-ci.

2. Substance selon la revendication 1, le ligand étant AICL.

3. Substance selon la revendication 1 ou 2, la maladie inflammatoire étant un trouble auto-immun.

4. Substance selon l'une quelconque des revendications 1 à 3, le trouble inflammatoire étant un trouble rhumatoïde inflammatoire, en particulier la polyarthrite rhumatoïde ou une affection associée à la polyarthrite rhumatoïde.

5. Substance de l'une quelconque des revendications 1 à 4, une substance étant utilisée qui comprend une séquence d'acides aminés, qui est choisie dans le groupe comprenant SEQ ID NO: 1 et SEQ ID NO: 2 de la liste de séquences annexée.

6. Substance de l'une quelconque des revendications 1 à 5, les anticorps étant des anticorps humanisés ou des fragments d'anticorps humanisés.

7. Substance de l'une quelconque des revendications 1 à 5, un anticorps anti-NKp80 étant utilisé qui est produit par des cellules d'hybridome, qui peut être obtenu par utilisation de l'ectodomaine de NKp80 soluble (NKp80-ED) pour l'immunisation d'animaux adaptés, isolement de cellules lymphoïdes productrices d'anticorps anti-NKp80 et fusion consécutive entre une cellule de myélome et la cellule lymphoïde productrice d'anticorps anti-NKp80.

8. Substance de l'une quelconque des revendications 1 à 7, un anticorps anti-NKp80 étant utilisé qui est produit par des cellules d'hybridome 5D12.

9. Substance de l'une quelconque des revendications 1 à 5, l'ectodomaine soluble de NKp80 étant utilisé.

10. Composition pharmaceutique comprenant en tant que substance active une substance capable de bloquer l'interaction entre NKp80 et son ligand sur des cellules myéloïdes, en association avec un véhicule pharmaceutiquement acceptable, la substance étant choisie dans le groupe comprenant un anticorps anti-NKp80, qui est produit par des cellules d'hybridome 5D12, NKp80 soluble, ou des fragments fonctionnels de ceux-ci.

11. Composition pharmaceutique selon la revendication 10, comprenant en tant que substance active un anticorps anti-NKp80 humanisé.

12. Procédé pour moduler *in vitro* l'activité de cellules exprimant NKp80 et/ou AICL, **caractérisé en ce que** lesdites cellules sont mises en contact respectivement avec AICL ou des fragments de celui-ci, et/ou avec NKp80 ou des fragments de celui-ci.

13. Procédé de la revendication 12, les cellules exprimant NKp80 étant des cellules NK.

14. Procédé de la revendication 12 ou 13, les cellules exprimant AICL étant des cellules myéloïdes, en particulier des cellules myéloïdes malignes.

15. Procédé pour cribler des composés adaptés pour être utilisés pour le traitement ou la prévention de maladies auto-immunes, **caractérisé en ce que** les substances adaptées pour être utilisées pour la prévention ou le traitement de telles maladies sont choisies sur la base de leur propriété de blocage de l'interaction entre NKp80 ou des fragments de celui-ci et AICL ou des fragments de celui-ci.


### SEQUENCE LISTING

<110> Eberhard-Karls-university Tubingen, University Hospital
<120> Methods for blocking the interaction between NKp80 and its ligands
<130> 5402P378
<160> 10
<170> PatentIn version 3.1
<210> 1
   <211> 231
   <212> PRT
   <213> Homo sapiens
<400> 1
   Met Gln Asp Glu Glu Arg Tyr Met Thr Leu Asn Val Gln Ser Lys Lys
   1 5 10 15
   Arg Ser Ser Ala Gln Thr Ser Gln Leu Thr Phe Lys Asp Tyr Ser Val
   20 25 30
   Thr Leu His Trp Tyr Lys Ile Leu Leu Gly Ile Ser Gly Thr Val Asn
   35 40 45
   Gly Ile Leu Thr Leu Thr Leu Ile Ser Leu Ile Leu Leu Val Ser Gln
   50 55 60
   Gly Val Leu Leu Lys Cys Gln Lys Gly Ser Cys Ser Asn Ala Thr Gln
   65 70 75 80
   Tyr Glu Asp Thr Gly Asp Leu Lys Val Asn Asn Gly Thr Arg Arg Asn
   85 90 95
   Ile Ser Asn Lys Asp Leu Cys Ala Ser Arg Ser Ala Asp Gln Thr Val
   100 105 110
   Leu Cys Gln Ser Glu Trp Leu Lys Tyr Gln Gly Lys Cys Tyr Trp Phe
   115 120 125
   Ser Asn Glu Met Lys Ser Trp Ser Asp Ser Tyr Val Tyr Cys Leu Glu
   130 135 140
   Arg Lys Ser His Leu Leu Ile Ile His Asp Gln Leu Glu Met Ala Phe
   145 150 155 160
   Ile Gln Lys Asn Leu Arg Gln Leu Asn Tyr Val Trp Ile Gly Leu Asn
   165 170 175
   Phe Thr Ser Leu Lys Met Thr Trp Thr Trp Val Asp Gly Ser Pro Ile
   180 185 190
   Asp Ser Lys Ile Phe Phe Ile Lys Gly Pro Ala Lys Glu Asn Ser Cys
   195 200 205
   Ala Ala Ile Lys Glu Ser Lys Ile Phe Ser Glu Thr Cys Ser Ser Val
   210 215 220
   Phe Lys Trp Ile Cys Gln Tyr
   225 230
<210> 2
   <211> 163
   <212> PRT
   <213> Homo sapiens
<400> 2
   Lys Cys Gln Lys Gly Ser Cys Ser Asn Ala Thr Gln Tyr Glu Asp Thr
   1               5                   10                  15
   Gly Asp Leu Lys Val Asn Asn Gly Thr Arg Arg Asn Ile Ser Asn Lys
               20                  25                  30
   Asp Leu Cys Ala Ser Arg Ser Ala Asp Gln Thr Val Leu Cys Gln Ser
           35                  40                  45
   Glu Trp Leu Lys Tyr Gln Gly Lys Cys Tyr Trp Phe Ser Asn Glu Met
       50                  55                  60
   Lys Ser Trp Ser Asp Ser Tyr Val Tyr Cys Leu Glu Arg Lys Ser His
   65                  70                  75                  80
   Leu Leu Ile Ile His Asp Gln Leu Glu Met Ala Phe Ile Gln Lys Asn
                   85                  90                  95
   Leu Arg Gln Leu Asn Tyr Val Trp Ile Gly Leu Asn Phe Thr Ser Leu
               100                 105                 110
   Lys Met Thr Trp Thr Trp Val Asp Gly Ser Pro Ile Asp Ser Lys Ile
           115                 120                 125
   Phe Phe Ile Lys Gly Pro Ala Lys Glu Asn Ser Cys Ala Ala Ile Lys
       130                 135                 140
   Glu Ser Lys Ile Phe Ser Glu Thr Cys Ser Ser Val Phe Lys Trp Ile
   145                 150                 155                 160
   Cys Gln Tyr
<210> 3
   <211> 149
   <212> PRT
   <213> Homo sapiens
<400> 3
   Met Met Thr Lys His Lys Lys Cys Phe Ile Ile Val Gly Val Leu Ile
   1 5 10 15
   Thr Thr Asn Ile Ile Thr Leu Ile Val Lys Leu Thr Arg Asp Ser Gln
   20 25 30
   Ser Leu Cys Pro Tyr Asp Trp Ile Gly Phe Gln Asn Lys Cys Tyr Tyr
   35 40 45
   Phe Ser Lys Glu Glu Gly Asp Trp Asn Ser Ser Lys Tyr Asn Cys Ser
   50 55 60
   Thr Gln His Ala Asp Leu Thr Ile Ile Asp Asn Ile Glu Glu Thr Asn
   65 70 75 80
   Phe Leu Arg Arg Tyr Lys Cys Ser Ser Asp His Trp Ile Gly Leu Lys
   85 90 95
   Met Ala Lys Asn Arg Thr Gly Gln Trp Val Asp Gly Ala Thr Phe Thr
   100 105 110
   Lys Ser Phe Gly Met Arg Gly Ser Glu Gly Cys Ala Tyr Leu Ser Asp
   115 120 125
   Asp Gly Ala Ala Thr Ala Arg Cys Tyr Thr Glu Arg Lys Trp Ile Cys
   130 135 140
   Arg Lys Arg Ile His
   145
<210> 4
   <211> 124
   <212> PRT
   <213> Homo sapiens
<400> 4
   Lys Leu Thr Arg Asp Ser Gln Ser Leu Cys Pro Tyr Asp Trp Ile Gly
   1 5 10 15
   Phe Gln Asn Lys Cys Tyr Tyr Phe Ser Lys Glu Glu Gly Asp Trp Asn
   20 25 30
   Ser Ser Lys Tyr Asn Cys Ser Thr Gln His Ala Asp Leu Thr Ile Ile
   35 40 45
   Asp Asn Ile Glu Glu Thr Asn Phe Leu Arg Arg Tyr Lys Cys Ser Ser
   50 55 60
   Asp His Trp Ile Gly Leu Lys Met Ala Lys Asn Arg Thr Gly Gln Trp
   65 70 75 80
   Val Asp Gly Ala Thr Phe Thr Lys Ser Phe Gly Met Arg Gly Ser Glu
   85 90 95
   Gly Cys Ala Tyr Leu Ser Asp Asp Gly Ala Ala Thr Ala Arg Cys Tyr
   100 105 110
   Thr Glu Arg Lys Trp Ile Cys Arg Lys Arg Ile His
   115 120
<210> 5
   <211> 20
   <212> DNA
   <213> Artificial Sequence
<220>
   <223> Primer
<400> 5
   cggctaccac atccaaggaa 20
<210> 6
   <211> 18
   <212> DNA
   <213> Artificial Sequence
<220>
   <223> Primer
<400> 6
   gctggaatta ccgcggct 18
<210> 7
   <211> 20
   <212> DNA
   <213> Artificial sequence
<220>
   <223> Primer
<400> 7
   ttcagtgacg ttgcactggt 20